# EUROPÄISCHE PATENTSCHRIFT

(11) **EP 1 602 387 B1**
(45) Veröffentlichungstag und Bekanntmachung des Hinweises auf die Patenterteilung: **14.03.2007**
(21) Anmeldenummer: 04013163.3
(22) Anmeldetag: 03.06.2004
(51) Int. Cl.: A61M 1/36, A61M 1/34

(54) **Vorrichtung zur Entfernung von bakteriellen Lipopolysacchariden oder/und Lipoteichonsäuren aus proteinhaltigen Flüssigkeiten sowie Verwendung zur Herstellung eines Mittels zur Entfernung dieser Stoffe**
Device for removal of bacterial lipopolysaccharides and/or lipoteichonic acids from liquids containing proteins and use for the manufacture of means for the removal of these compounds
Dispositif d'elimination des lipopolysaccharides et/ou de l'acide lipoteichoique des liquides contenant des proteines ainsi que son utlilisation pour fabriquer un moyen pour leurs elimination

(43) Veröffentlichungstag der Anmeldung: 07.12.2005
(73) Patentinhaber: B. Braun Medizintechnologie GmbH, 34212 Melsungen (DE)
(72) Erfinder: Seidel, Dietrich, Prof. Dr. Dr. med., 82340 Feldafing (DE); Boos, Karl-Siegfried, Prof. Dr. Dr. rer. nat., 82131 Gauting (DE)
(74) Vertreter: Böhm, Brigitte

(56) Entgegenhaltungen:
- EP-A- 1 002 566
- EP-A- 1 316 355
- DE-A- 3 926 539
- DE-A- 19 515 554
- DE-A- 19 740 770
- DE-A- 19 938 394
- US-A- 5 403 917

## Beschreibung

Die vorliegende Erfindung betrifft eine Vorrichtung zur Entfernung von Lipoteichonsäuren oder zur Entfernung von Lipopolysacchariden (LPS, Endotoxinen) und Lipoteichonsäuren (Lipoteichoic Acids, LTA) aus proteinhaltigen Flüssigkeiten, insbesondere aus Blut oder Plasma, und erläutert die Verwendung einer solchen Vorrichtung zur Behandlung von Patienten mit Erkrankungen, welche hervorgerufen sind durch die Invasion gram-negativer oder/und gram-positiver Bakterien.

Beim Vorliegen einer Bakteriämie im Blutkreislauf des Menschen können oftmals schwere, sogenannte septische Komplikationen bzw. Krankheitsverläufe entstehen.

Bei 50 bis 95 % der Patienten mit schwerer Sepsis oder septischem Schock muß trotz aller therapeutischen Maßnahmen mit einem letalen Ausgang gerechnet werden (Stone, R., Science (1994) 264: 365-367). Die Inzidenz der Sepsis hat in den vergangenen Jahren signifikant zugenommen. Die Gründe hierfür liegen unter anderem in der zunehmenden diagnostischen und therapeutischen Anwendung von Kathetern, Endoskopen, Implantationen von Prothesen, großen chirurgischen Eingriffen, im Einsatz von immunsuppresiven Medikamenten, in der Zunahme an älteren Patienten und in der steigenden Resistenz der Bakterien gegenüber Antibiotika. Infektionen der Intensivpatienten werden heute vorwiegend durch resistente Bakterien verursacht (Vincent, I.L., JAMA (1995) 274: 639-644).

Verantwortlich für die Pathogenität der gram-negativen Bakterien sind vor allem die während und nach der Bakteriolyse und medikamentösen Antibiose freigesetzten Endotoxine (Lipopolysaccharide, LPS).

Lipopolysaccharide sind als Bestandteile der äußeren Membran gram-negativer Bakerien aus drei strukturell unterschiedlichen Regionen aufgebaut. Der Träger der toxischen Eigenschaften ist das Lipid-A. Diese Subregion mit einem Molekulargewicht von 2000 Dalton besteht aus einem phosphorylierten D-Glucosamin-Disaccharid, an das ester- und amidartig mehrere langkettige Fettsäuren gebunden sind (Bacterial Endotoxic Lipopolysaccharides, Morrison, D.C., Ryan, J.L. eds., 1992, CRC Press). Die bakteriellen Lipopolysaccharide (LPS) gelten als initiierende Mediatoren und wichtigste Toxine in der Pathogenese des septischen Schocks. Das klinische Bild der Sepsis korreliert häufig mit der Höhe der LPS-Konzentration im Blut der Patienten (Nitsche, D. et al., Intensive Care Med., 12 Suppl., 1986, 185 ff). Die Lipopolysaccharide stimulieren die als Makrophagen bezeichneten Fresszellen im Organismus zur Produktion und Freisetzung von Entzündungsmediatoren wie TNF α und Interleukinen.

Die Entfernung insbesondere von LPS, jedoch auch des körpereigenen Entzündungsmediators TNF α war daher der bisherige Ansatz zur Behandlung von Sepsis-Kranken.

Allerdings hat man festgestellt, dass nicht nur die LPS der gram-negativen Bakterien eine große Rolle spielen, sondern dass auch Infektionen durch gram-positive Bakterien, welche keine LPS enthalten, zu septischen Komplikationen führen, wobei insbesondere die gefährlichen Krankenhauskeime wie Staphylococcus aureus sich als besonders tückisch erwiesen haben.

Nahezu 50 % der septischen Patienten haben eine Infektion mit gram-positiven Bakterien. Etwa 30 % der Patienten leiden unter einer gemischten bakteriellen Infektion.

Die Toxine der gram-positiven Bakterien, die Lipoteichonsäuren (Lipoteichoic Acids, LTA) befinden sich ebenfalls in der Zellwand und werden bei der Lyse der Bakterien feigesetzt. Die LTA bestehen aus Glycerin- oder Ribit-Polymeren, die über 1→3 Phosphodiester-Bindungen mit Glykolipiden oder Phosphatidyl-Glykolipiden verknüpft sind. Zusätzlich tragen die Polyglycerin- bez. Ribit-Ketten Glucose und/oder N-Acetylglucosamin-Reste. Die LTA stimulieren analog LPS Monozyten und andere immunkompetente Zellen zur Cytokin-Produktion (Mattson, T., FEMS Immunol. Med. Microbiol. (1993) 7: 281-288). Die deletäre biologische Mediatorkaskade wird ähnlich wie bei LPS über lösliche und membranständige Rezeptoren, die sich im Blutkreislauf befinden, vermittelt bzw. angestoßen (Cleveland M.G., Infect. Immun. (1996) 64: 1906-19121.

In die Blutbahn eingeschwemmtes LTA bindet an die Zellen des Monozyten-Makrophagen-Systems und stimuliert diese zu einer gesteigerten Produktion und Freisetzung von Mediatoren (Cytokinen). Als initialer Mediator und potenter proinflammatorischer Stimulus wird zunächst der Tumor-Nekrose-Faktor α synthetisiert und in die Blutbahn sezerniert. Die biologisch wirksame Form des TNF α besteht aus einem Aggregat dreier identischer Polypeptidketten (157 Aminosäuren, Molekulargewicht: 17,4 × 10³ Dalton; Ziegler, E.J., N. Engl. J. Med. 318 (1999) 1533 ff). Die nachfolgende biologische Signalverstärkung über Interleukine, Leukotriene, Prostaglandine und Interferone (Mediatorkaskade) kann schließlich schwere Störungen der Homöostase verschiedener biologischer Regelkreise und Organsysteme, wie beispielsweise das Krankheitsbild des septischen Schocks hervorrufen.

Somit nehmen die Lipopolysaccharide (LPS) als initiierende Toxine gram-negativer Bakterien und die Lipoteichonsäuren (LTA) als hochpotente Pyrogene gram-positiver Bakterien eine Schlüsselrolle hinsichtlich der Pathogenese einer Sepsis ein.

Neben der Herdsanierung durch chirurgische Maßnahmen und der Gabe von Antibiotika haben neue therapeutische Ansätze, die über die Sanierung der Sespisquelle hinausgehen, wie die Plasmapherese, die Gabe von Immunglobulinen und Antikörpern gegen LPS (Bone, R.C., Crit. Care Med. (1995) 23: 994-1000) oder TNF α (Fisher, C.J., N. Engl. J. Med. (1996) 334: 1697-1702) die Prognose für einen septischen Patienten nicht wesentlich verbessern können.

Die Plasmapherese (Plasmaaustauschbehandlung) ist ein unselektives und ineffizientes Verfahren. Neben der Elimination von Toxinen und proinflammatorischen Cytokinen werden dem Patienten auch protektive, anti-inflammatorische Mediatoren entzogen. Ein einzelner Therapiezyklus benötigt ein Austauschvolumen von ca. 12 Litern an Plasma. Daraus ergibt sich ein Bedarf von ca. 50 Spendern, was ein erhöhtes Risiko bezüglich zusätzlicher Infektionen und allergischer Reaktionen mit sich bringt.

Antikörper-Therapieverfahren weisen ebenfalls schwerwiegende Mängel und Nachteile auf. Die Kosten für die technisch aufwändige Gewinnung, Reinigung und Charakterisierung der entsprechenden Antikörper sind sehr hoch und es besteht die Gefahr der allergischen Gegenreaktion (neutralisierende Immunantwort) des Körpers auf die Antikörper. Bezüglich der LPS-Antikörper können die hohen Raten von Therapieversagen unter anderem auf die zu geringe Spezifität bzw. Affinität zwischen den sehr heterogenen LPS-Molekülen und den verwendeten mono- bzw. polyklonalen Antikörpern zurückgeführt werden. In diesem Zusammenhang mußten klinische Multi-Zentren-Studien vorzeitig abgebrochen werden (Luce, J.M., Crit. Care Med. 21 (1993), 1233 ff).

Eine andere Vorgehensweise zur Neutralisation bzw. Elimination pathogener Blutbestandteile besteht in der Behandlung von Vollblut oder Plasma in einem extrakorporalen Perfusionssystem unter Verwendung entsprechend geeigneter Adsorbermaterialien.

Folgende Adsorbermaterialien wurden für eine extrakorporale Elimination von Lipopolysacchariden aus Vollblut und/oder Plasma als potentiell geeignet offenbart:

Poröse Trägermaterialien mit immobilisiertem Polymyxin B sind in US 4,576,928; DE 39 32 971 beschrieben. Die klinische Anwendung dieser Affinitätsträger ist jedoch sehr problematisch, da der Ligand Polymyxin B schwere nephro- und neurotoxische Schäden bei seiner Freisetzung in die Blutbahn hervorruft.

Die in DE 41 13 602 A1 offenbarten polyethylenimin-modifizierten Perlcellulosen besitzen eine geringe Bindungskapazität für LPS. Bei ihrer Verwendung in einem extrakorporalen Perfusionssystem würde daher das medizinisch-tolerierbare extrakorporale Totvolumen überschritten.

DE 197 40 770 offenbart Mikrofiltrationsfilterschichten zur Abtrennung von Endotoxinen aus flüssigen Medien, insbesondere Wasser, Proteinlösungen oder Parenteralien.

DE 199 38 394 beschreibt Immunoadsorber zum spezifischen Entfernen bakterieller Endotoxine aus Flüssigkeiten, wässrigen, kristallinen und/oder kolloidalen Lösungen, Emulsionen und Körperflüssigkeiten und/oder aus Körperflüssigkeiten gewonnenen Flüssigkeiten, wie beispielsweise Serum oder Dialysat.

Das für die Elimination von LPS und TNF α beschriebene H.E.L.P.-Verfahren (DE 44 35 612 A1) ist komplex und stellt daher hohe Anforderungen an seinen Betrieb auf einer Intensivstation. Das System bemötigt ein sehr großes extrakorporales Totvolumen und ist daher aus hämodynamischen Gründen weniger vorteilhaft für den zu behandlenden Patienten. Ein weiterer Nachteil dieses Plasmaperfusionsverfahrens besteht darin, dass neben LPS und TNF α auch das für die plasmatische Gerinnung essentielle Fibrinogen sehr effektiv eliminiert wird. Je nach Ausgangskonzentration von Fibirinogen ist damit die Anwendung des H.E.L.P.-Verfahrens auf maximal 2 bis 3 konsekutive Behandlungen beschränkt, was für eine effektive Behandlung des Patienten in vielen Fällen ungenügend ist.

Gegenüber dem in DE 195 15 554 A1 im Beispiel 2 beschriebenen Anionenaustauschermaterial besitzen die in der vorliegenden Erfindung aufgeführten Anionenaustauschermaterialien auf Hohlfaserbasis den Vorteil, dass diese keine Glukane freisetzen können. Letztere stören den analytischen Nachweis der bakteriellen Endotoxine im Plasma des Patienten. Darüber hinaus können Glukane eine unerwünschte Hämolyse sowie eine unerwünschte Immunstimulation im Patienten hervorrufen.

Der vorliegenden Erfindung lag die Aufgabe zugrunde, eine Behandlungsmöglichkeit und eine dafür geeignete Vorrichtung bereitzustellen, mit denen die Nachteile der bisher angewandten Verfahren vermieden werden können, und die eine effektive Behandlung von Sepsispatienten im Allgemeinen erlauben, unabhängig von der Art der verursachenden Bakterien.

Diese Aufgabe wird erfindungsgemäß gelöst durch eine Vorrichtung zur Entfernung von bakteriellen Lipoteichonsäuren oder zur Entfernung von bakteriellen Lipopolysacchariden und Lipoteichonsäuren aus Blut oder Plasma in einem extrakorporalen Perfusionssystem, welche in einem in das Perfusionssystem integrierten Gehäuse ein zur selektiven Entfernung von bakteriellen Lipopolysacchariden und Lipoteichonsäuren geeignetes chemisch modifiziertes Hohlfasermaterial aus Polyamid, Polysulfon, Polyether, Polyethylen, Polypropylen, Polyester oder Derivaten oder/und Mischungen solcher Polymere umfaßt, auf welches Tentakeln aufpolymerisiert wurden, die Anionenaustauschergruppierungen aus synthetischen oder/und halbsynthetischen oder/und natürlichen Polykationenketten in linearer oder verzweigter Form umfassen, wobei die Vorrichtung ein Totvolumen von < 150 ml und vorzugsweise ein solches von 80 bis 130 ml aufweist.

Die erfindungsgemäße Vorrichtung ist dabei so ausgestaltet, dass das durch eine erste Öffnung des Geäuses eintretende Blut oder Plasma das Hohlfasermaterial passieren muss, bevor es durch eine zweite Öffnung des Gehäuses austritt und dem weiteren Perfusions-Kreislauf zugeführt wird.

Un eine derartige Vorrichtung zur Adsorptionsapherese nutzen zu können, müssen unter anderem folgende Voraussetzungen erfüllt sein:
1. Die Elimination der Pathogene sollte möglichst selektiv und effizient erfolgen.
2. Die Bindungskapazität der verwendeten Adsorbentien sollte optimalen praktischen Anforderungen genügen.
3. Die Adsorbentien müssen ohne Verlust oder Veränderung ihrer Eigenschaften mit Hitze oder gamma-Stahlen sterilisierbar sein.
4. Die Adsorbentien sollten eine ausreichend hohe Durchflussgeschwindigkeit im Bereich bis zu 200 ml/min. erlauben.
5. Das Eliminationsverfahren muss die medizinisch-erforderliche Bio- bzw. Hämokompabilität aufweisen und darf keine physiologischen Regelkreise und Schutzmechanismen, wie beispielsweise das Immun-, Komplement- oder Gerinnungssystem beeinträchtigen.

Der Fachmann kann gemäß diesem Anforderungsprofil geeignete HohlfaserAdsorptionsmaterialien auffinden. Im Prinzip können Hohlfasermaterialien verwendet werden, welche aus Polyamid, Polysulfon, Polyether, Polyethylen, Polypropylen, Polyester oder Derivaten oder/und Mischungen solcher Polymere hergestellt wurden. In besonders bevorzugter Ausführungsform der Erfindung bestehen die Hohlfasern aus Nylon.

Erfindungsgemäß geeignete Hohlfasermaterialien basieren auf Affinitätsmembranen, wie sie beispielsweise im U.S. Patent Nr. 5,053,133, 5,766,908, in der WO96/22316 beschrieben sind. Diese Membrangrundmaterialien können nach an sich bekannten Methoden und insbesondere durch Pfropfpolymerisation modifiziert werden, beispielhaft sei in diesem Zusammenhang auf die WO96/22316 hingewiesen, in der ein entsprechendes Verfahren beschrieben ist. Andere Derivatisierungsverfahren für entsprechende Polymermaterialien, welche zur Herstellung von Hohlfaseradsorptionsmaterialien geeignet sind, sind dem Fachmann ebenfalls bekannt und im Rahmen der vorliegenden Erfindung anwendbar.

Die erfindungsgemäßen Hohlfasermaterialien sind vorzugsweise in der erfindungsgemäßen Vorrichtung in solcher Weise angeordnet, dass sie vom Blut bzw. Plasma effektiv durchströmt werden, sodass eine maximale Benetzung der Hohlfasermembran erfolgt und somit eine bestmögliche Adsorption der LPS und LTA an die Hohlfaseradsorptionsmaterialien stattfinden kann. Bevorzugte Anordnungen der Hohlfasermaterialien in einer erfindungsgemäßen Vorrichtung sind in der WO98/57733, der WO98/33581, der WO98/19777 oder der WO98/28064 offenbart, eine besonders bevorzugte Anordnung ist darüber hinaus in der EP 1 002 566 beschrieben.

Neben den Hohlfasermaterialien kann eine erfindungsgemäße Vorrichtung zusätzliche Materialien, wie z.B. Flachmembranen, zwischen den Hohlfasern aufweisen, wodurch die Adsorptions- und Trennleistung oftmals noch erhöht werden kann. Entsprechende Adsorberanordnungen sind in der EP 0 956 147 beschrieben.

Sowohl die in den oben genannten Schriften offenbarten Materialien als auch die Modifizierungsverfahren und letztendlichen Anordnungen in Adsorbern können in der erfindungsgemäßen Vorrichtung Einsatz finden.

Um die Entfernung von LPS und LTA aus dem Blut oder Plasma besonders effizient zu gestalten, werden in der Vorrichtung Hohlfasern eingesetzt, welche chemisch in solcher Weise modifiziert sind, dass die geladenen LPS und LTA Moleküle besonders gut an das Hohlfasermaterial gebunden und damit aus dem Blut oder Plasma entfernt werden. Bevorzugt erfolgt hierzu eine chemische Modifikation des Hohlfasermaterials, besonders günstig hat sich für eine solche Modifikation eine Pfropfpolymerisation (siehe oben) erwiesen, bei der auf das Hohlfasermaterial Anionenaustauschergruppierungen aufgepfropft werden, welche gute Bindefähigkeit für LPS oder/und LTA zeigen. Derartige Antionenaustauschergruppierungen sind als längere Ketten mit einer Vielzahl von kationischen Gruppen, als sogenannte Tentakeln, ausgestaltet. Derartige tentakelartige Fortsätze auf dem Grundmaterial sind fähig, mehrere LPS bzw. LTA-Moleküle zu binden, wodurch die Effizienz des Hohlfasermaterials nochmals gesteigert werden kann. Für diese Modifikation des Hohlfasermaterials mittels Tentakeln werden synthetische oder/und halbsynthetische oder/und natürliche Polykationenketten eingesetzt, wobei diese Ketten in linearer oder verzweigter Form vorliegen können. Die erfindungsgemäßen Hohlfasermaterialien sind durch Kationen- bzw. Polykationenketten, welche tertiäre oder/und quartäre Amine aufweisen, modifiziert.

Bevorzugte Anionenaustauschergruppen auf den Hohlfasermaterialien schließen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- und Di- oder Trialkylammoniumaryl-Reste ein. Des Weiteren sind Polymere aus positiv geladenen oder tertiäre oder quartäre Aminogruppen enthaltenden Aminosäuren, wie Polylysin, Polyarginin oder Polyhistidin oder Mischpolymere oder Derivate hiervon im Rahmen der Erfindung als Anionenaustauschermaterialien geeignet, ebenso Polyethylenimin.

In ganz besonders bevorzugten Ausführungsformen der Erfindung enthält die Vorrichtung ein mit Diethylaminoalkyl- bzw. Diethylaminoaryl-Resten modifiziertes Polyamid-Hohlfasermaterial, insbesondere Diethylaminoethyl-Polyamid.

Des Weiteren ist es bevorzugt, die Vorrichtung in solcher Weise auszugestalten, daß sie als auswechselbare Filterkartusche für ein bestehendes Perfusionssytem eingesetzt werden kann. Diese kann in das Perfusionssystem leicht eingesetzt werden und kann aufgrund der hohen Bindungskapazität und -spezifität des verwendeten Hohlfasermatierals mit einem geringen Volumen auskommen, wochurch sich ein deutlich verringertes Totvolumen im Vergleich zu bisher beschriebenen Systemen erzielen läßt. Überraschenderweise kann eine erfindungsgemäße Vorrichtung als Kartusche mit den beispielhaften Abmessungen 12 cm Länge und 5 cm Durchmesser hergestellt werden, welche äußerst effektiv in einem extrakorporalen Perfusionssystem eingesetzt werden kann. Eine solche beispielhafte Kartusche weist ein Tot-Volumen von lediglich ca. 115 ml auf. Es ist daher im Rahmen der vorliegenden Erfindung besonders bevorzugt, die Kartuschen so zu dimensionieren, dass das Tot-Volumen < 150 ml und vorzugsweise 80 bis 130 ml beträgt.

Da gezeigt werden konnte, dass solche Filterkartuschen effektiv und selektiv LPS und LTA entfernen können, ergeben sich bereits aus diesem geringen Tot-Volumen überraschende und erhebliche Vorteile gegenüber dem Stand der Technik zusätzlich zu der erfindungsgemäß neu aufgefundenen Möglichkeit, LPS und LTA gleichzeitig in einer Anwendungsform zu entfernen. Nach Behandlungsende und Reinigung der Apparatur oder auch zur Durchführung einer verlängerten (kontinuierlichen) Blut- oder Plasmaperfusion kann einfach eine neue Kartusche eingesetzt werden.

Im Rahmen der vorliegenden Erfindung konnte festgestellt werden, dass die entsprechenden Hohlfasermaterialien sowohl LPS, als auch LTA bei physiologischem pH-Wert mit hoher Selektivität und Kapazität adsorptiv aus Vollblut oder/und Blutplasma eliminieren.

Weiterhin wurde festgestellt, dass auch bei physiologischem pH-Wert nur eine geringe und von der Zusammensetzung unschädliche Menge ans Blut bzw. Plasmaproteinen adsorbiert werden. Insbesondere Fibrinogen wird im Rahmen der vorliegenden Erfindung nur in ganz geringer Weise (< 2 %) aus dem Patientenblut entfernt, sodass die natürliche Gerinnungskaskade bei Einsatz der erfindungsgemäßen Vorrichtung praktisch nicht beeinträchtigt wird. Aufgrund der außerordentlich hohen Bindekapazität für sowohl LPS auf der einen und LTA auf der anderen Seite, ermöglicht die erfindungsgemäße Vorrichtung erstmals die Behandlung einer Sepsis unabhängig davon, ob gram-positive oder gram-negative Bakterien die Erkrankung verursachen, oder sogar eine gemischt-bakterielle Infektion vorliegt.

Ein weiterer Gegenstand der Erfindung ist folglich die Verwendung der beschriebenen und in der erfindungsgemäßen Vorrichtung enthaltenen chemisch modifizierten Hohlfasermaterialen zur Herstellung eines Mittels zur Entfernung von bakteriellen Lipoteichonsäuren oder zur gemeinsamen Entfernung von bakteriellen Lipoteichonsäuren und Lipopolysacchariden aus Körperflüssigkeiten, insbesondere aus Blut oder Plasma, wobei die Hohlfasermaterialien ausgewählt sind aus der Gruppe umfassend Polyamide, Polysulfone, Polyether, Polyethylen, Polypropylen oder Polyester sowie Derivate und/oder Mischungen dieser Materialien, und auf diese Hohlfasermaterialien Tentakeln aufpolymerisiert wurden, welche Anionenaustauschergruppierungen umfassen, welche aus synthetischen und/oder halbsynthetischen oder/und natürlichen Polykationenketten bestehen, welche in linearer oder verweigter Form vorliegen.

Die erfindungsgemäß eingesetzten Hohlfasermaterialien, welche ausgewählt sind aus der Gruppe umfassend Polyamide, Polysulfone, Polyether, Polyethylen, Polypropylen oder Polyester sowie Derivate oder/und Mischungen dieser Materialen, ermöglichen eine effektive Eliminierung von LPS oder/und LTA aus dem Blutkreislauf eines Patienten und können daher vorteilhaft in einer Adsorptionsaphereseapparatur eingesetzt werden. In der Regel wird in einer solchen Apparatur zuerst eine Trennung von Vollblut in Plasma und korpuskuläre Blutbestandteile stattfinden, das Plasma wird dann in der Folge über das Adsorptionsmaterial geleitet und darauffolgend wieder mit den korpuskulären Blutbestandteilen versetzt.

Die erfindungsgemäß als Adsorbentien verwendeten Hohlfasermaterialien sind chemisch modifiziert in solcher Weise, dass eine optimale LPS- bzw. LTA-Adsorption hieran stattfinden kann. Besonders bevorzugt sind die Hohlfasern über Pfropfpolymerisation modifiziert, wobei das Aufpfropfen von Anionenaustauschergruppierungen in Form von sogenannten Tentakeln, also kettenförmigen, verzweigten Molekülen mit möglichst vielen Anionenaustauschergruppierungen, vorteilhaft ist.

Ein besonders bevorzugtes Hohlfasermaterial ist ein durch DEAE modifiziertes Polyamid.

Wie oben im Hinblick auf die erfindungsgemäße Vorrichtung bereits ausgeführt, ist es vorteilhaft, das Mittel in Form eines Filters auszugestalten, der möglichst in Form einer Einmalkartusche leichte Handhabbarkeit und sicheren Einsatz gewährleistet.

Die erfindungsgemäß hergestellten Mittel können besonders vorteilhaft zur Behandlung von Patienten mit Sepsis und insbesondere mit septischem Schock eingesetzt werden, da sie aufgrund der guten Selektivität und Spezifität für LPS und LTA die bakteriellen Pyrogene aus dem Patientenblut entfernen und dadurch eine weitere Stimulierung der Entzündungsmediator-Kaskade unterbleibt. Durch die hohe Spezifität für sowohl LPS als auch LTA wird eine Behandlung von Patienten mit der gleichen Appartur bzw. dem gleichen Mittel ermöglicht, unabhängig davon, ob sie an einer durch gram-positive oder gram-negative Bakterien oder von beiden Arten verursachte Sepsis leiden.

Die entsprechende Verwendung der erfindungsgemäßen Vorrichtung bzw. des erfindungsgemäß hergestellten Mittels zur Entfernung von LPS oder/und LTA aus Körperflüssigkeiten, insbesondere Blut oder Plasma, und insbesondere im Rahmen einer Behandlung von Sepsis bis hin zum septischen Schock ist daher in vorteilhafter weise möglich. Besonders vorteilhaft wird die erfindungsgemäße Vorrichtung bzw. das erfindungsgemäß hergestellte Mittel in einem extrakorporalen Perfusionssystem eingesetzt, was eine besonders effektive Entfernung der bakteriellen Pyrogene aus dem Patientenblut ermöglicht und zu einer außerordentlich erfolgreichen Behandlung der Patienten führt. Bei der Verwendung der Erfindungsgegenstandes wird des Weiteren sehr selektiv LPS oder/und LTA entfernt, wogegen körpereigene Proteine wenn überhaupt nur in sehr geringem Ausmaß entfernt werden bzw. eine solche Entfernung lediglich Proteine betrifft, welche leicht substituierbar sind und deren Entfernung für den ohnehin besonders geschwächten Sepsispatienten nicht merklich belastend ist.

Im Rahmen der vorliegenden Erfindung ist es ebenfalls möglich, die beschriebenen Adsorbermaterialien zur Isolierung und Anreicherung von LPS und LTA einzusetzen, eine derartige Verwendung einer erfindungsgemäßen Vorrichtung ist daher ein weiterer Gegenstand der vorliegenden Erfindung. Eine solche Isolierung und Anreicherung kann aus beliebigen Flüssigkeiten, vorzugsweise aus Blut oder Plasma, erfolgen, wobei dies zu beliebigen Zwecken, insbesondere jedoch zur Analyse oder/und Diagnose erfolgen kann.

Die Erfindung wird durch die folgenden Beispiele näher erläutert.

### Beispiel 1

### Adsorption und Elimination von LPS aus Humanplasma bei Perfusion durch ein erfindungsgemäßes Adsorbermodul

Das eingesetzte Adsorbermodul (Länge: 12 cm, Durchmesser: 5 cm) war mit Polyamid-Hohlfasern entsprechend EP 1 002 566 A2 gepackt. Die Oberfläche der Hohlfasern war erfindungsgemäß mit aufpolymerisierten und mit DEAE-Gruppen funktionalisierten Tentakeln modifiziert. Vor der Perfusion mit Humanplasma wurde das Modul zunächst mit 500 ml einer Ringer-Lösung (140 mmol/l NaCl; 2 mmol/l CaCl₂ und 4 mmol/l KCl) gewaschen bzw. konditioniert.

Zu 100 ml Humanplasma wurden 140 ng (entspricht ca. 1400 Endotoxin-Einheiten, EU) an radioaktivem ³H-LPS (E.coli K12 strain LCD 25) mit einer spezifischen Aktivität von 4 × 10⁵ dpm/µg zugegeben. Diese Mischung wurde bei Raumtemperatur mithilfe einer Schlauchpumpe und einer Flussrate von 20 ml/min über das erfindungsgemäße Hohlfasermodul gepumpt und Eluatfraktionen (5 ml) alle 15 Sekunden gewonnen. Die nachfolgende Flüssigszintillationsmessung der Radioaktivität ergab, dass in keiner der gewonnenen Fraktionen ³H-markiertes LPS nachgewiesen werden konnte.

Parallel erfolgte ein quantitativer Nachweis des LPS im Perfusat mifhilfe des chromogenen kinetischen Limulus-Amoebocyten-Lysat Tests (Fa. BioWhittaker). Auch dieses spurenanalytische Messverfahren ergab, dass das dem Humanplasma zugesetzte LPS im Perfusat nicht mehr nachweisbar war und daher vollständig eliminiert wurde.

### Beispiel 2

### Adsorption und Elimination von LTA aus Humanplasma bei Perfusion durch ein erfindungsgemäßes Adsorbermodul

Das eingesetzte Adsorbermodul (Länge: 12 cm, Durchmesser: 5 cm) war mit Polyamid-Hohlfasern entsprechend EP 1 002 566 A2 gepackt. Die Oberfläche der Hohlfasern war erfindungsgemäß mit aufpolymerisierten und mit DEAE-Gruppen funktionalisierten Tentakeln modifiziert. Vor der Perfusion mit Humanplasma wurde das Modul zunächst mit 500 ml einer Ringer-Lösung (140 mmol/l NaCl; 2 mmol/l CaCl₂ und 4 mmol/l KCl) gewaschen bzw. konditioniert.

100 ml Humanplasma wurden mit 200 µg LTA aus Streptococcus pyogenes (Fa. Sigma-Aldrich) versetzt und bei Raumtemperatur mithilfe einer Schlauchpumpe und einer Flussrate von 20 ml/min über das oben beschriebene Adsorbermodul gepumpt. Im 15 Sekunden Abstand wurden 5 ml Eluatfraktionen gewonnen und auf eine LTA-Aktivität hin untersucht.

Dies erfolgte mithilfe eines Vollblut-Stimulationstests. Hierzu wurde frisch gewonnenes, heparinisiertes Vollblut eines gesunden Spenders mit jeweils einem Aliquot der erhaltenen Eluatfraktionen im Verhältnis 6 : 1 (v/v) versetzt und bei Raumtemperatur für 24 Stunden inkubiert. Danach wurde nach Zentrifugation und Gewinnung der entsprechenden Plasmafraktion das Interleukin-6 (IL-6) mithilfe eines ELISA-Tests (Fa. Biosource) in allen gewonnenen bzw. inkubierten Eluatfraktionen quantitativ bestimmt.

Als Ergebnis wurde gefunden, dass keine der perfundierten Plasmafraktionen zu einer LTA-vermittelten Stimulation der IL-6 Biosynthese, d.h. zu einer gegenüber der Kontrollprobe (perfundiertes Aliquot einer Plasmaprobe ohne LTA-Zusatz) erhöhten IL-6 Konzentration führte. Damit wurde mithilfe dieses hochempfindlichen Bioassays zwar indirekt, aber hochsignifikant bewiesen, dass das erfindungsgemäß eingesetzte Adsorbermodul bakterielle Lipoteichonsäure aus Humanplasma vollständig eliminiert.

### Beispiel 3

### Simultane Adsorption und Elimination von LPS und LTA aus Humanplasma bei Perfusion durch ein erfindungsgemäßes Adsorbermodul

Das eingesetzte Adsorbermodul (Länge: 12 cm, Durchmesser: 5 cm) war mit Polyamid-Hohlfasern entsprechend EP 1 002 566 A2 gepackt. Die Oberfläche der Hohlfasern war erfindungsgemäß mit aufpolymerisierten und mit DEAE-Gruppen funktionalisierten Tentakeln modifiziert. Vor der Perfusion mit Humanplasma wurde das Modul zunächst mit 500 ml einer Ringer-Lösung (140 mmol/l NaCl; 2 mmol/l CaCl₂ und 4 mmol/l KCl) gewaschen bzw. konditioniert.

100 ml Humanplasma wurden mit 2000 EU an bakteriellem LPS (E.coli 055: B5 Endotoxin; Fa. BioWhittaker) und 100 *µ*g LTA (Streptococcus pyogenes; Fa. Sigma-Aldrich) versetzt.

Gemäß der Versuchsvorschrift in Beispiel 2 wurden entsprechende Perfusat-Fraktionen gewonnen und hinsichtlich der darin vorhandenen LTA- bzw. LPS-Aktivität mithilfe des beschriebenen Vollblut-Stimulationstests untersucht. Die Analyse ergab, dass in keinem der Perfusate eine LTA- bzw. LPS-Aktivität nachweisbar war.

## Patentansprüche

1. Verwendung von chemisch modifizierten Hohlfasermaterialien zur Herstellung eines Mittels zur Entfernung von bakteriellen Lipoteichonsäuren oder zur gemeinsamen Entfernung von bakteriellen Lipoteichonsäuren und Lipopolysacchariden aus Körperflüssigkeiten, insbesondere Blut oder Plasma, wobei die Hohlfasermaterialien ausgewählt sind aus der Gruppe umfassend Polyamide, Polysulfone, Polyether, Polyethylen, Polypropylen oder Polyester sowie Derivate und/oder Mischungen dieser Materialien, und auf diese Hohlfasermaterialien Tentakeln aufpolymerisiert wurden, welche Anionenaustauschergruppierungen umfassen, welche aus synthetischen und/oder halbsynthetischen oder/und natürlichen Polykationenketten bestehen, welche in linearer oder verzweigter Form vorliegen.

2. Verwendung nach Anspruch 1, **dadurch gekennzeichnet, dass** durch Propfpolymerisation modifizierte Hohlfasern verwendet werden.

3. Verwendung nach Anspruch 1 oder 2, **dadurch gekennzeichnet, dass** die aufpolymerisierten Anionenaustauschergruppierungen aus Polykationen-ketten bestehen, welche tertiäre und/oder quartäre Amine aufweisen.

4. Verwendung nach einem der Ansprüche 1 bis 3, **dadurch gekennzeichnet, dass** die Anionenaustauschergruppen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- oder Di- oder Trialkylammoniumaryl-Reste, Polymere aus positiv geladenen oder tertiäre oder quartäre Aminogruppen enthaltenden Aminosäuren, wie Polylysin, Polyarginin oder Polyhistidin oder Mischpolymere oder Derivate hiervon oder Polyethylenimin sind.

5. Verwendung nach einem der vorhergehenden Ansprüche, **dadurch gekennzeichnet, dass** das Hohlfasermaterial ein durch die Diethylaminoethylgruppen modifiziertes Polyamid ist.

6. Verwendung nach einem der Ansprüche 1 bis 5, **dadurch gekennzeichnet, dass** das Mittel in Form eines Filtermoduls ausgestaltet wird.

7. Verwendung nach Anspruch 6, **dadurch gekennzeichnet, dass** das Filtermodul ein Totvolumen von < 150 ml und insbesondere von 80-130 ml aufweist.

8. Verwendung nach Anspruch 6 oder 7, **dadurch gekennzeichnet, dass** das Mittel eine Durchflussgeschwindigkeit von bis zu 200 ml/Minute erlaubt.

9. Verwendung nach einem der Ansprüche 1 bis 8, **dadurch gekennzeichnet, dass** das Mittel zur Anwendung in einem Plasmaperfusionssystem ausgestaltet ist.

10. Verwendung nach einem der vorhergehenden Ansprüche, **dadurch gekennzeichnet, dass** das Mittel zur Behandlung einer durch Zellwandbestandteile gram-negativer und/oder gram-positiver Bakterien verursachten Erkrankung geeignet ist.

11. Verwendung nach einem der vorhergehenden Ansprüche, **dadurch gekennzeichnet, dass** das Mittel im Rahmen einer Behandlung von Sepsis, insbesondere septischem Schock, eingesetzt werden kann.

12. Vorrichtung zur Entfernung von bakteriellen Lipoteichonsäuren oder zur Entfernung von bakteriellen Lipopolysacchariden und Lipoteichonsäuren aus Blut oder Plasma in einem extrakorporalen Perfusionsystem, **dadurch gekennzeichnet, dass** in einem in das Perfusionssystem integrierten Gehäuse ein zur selektiven Entfernung von bakteriellen Lipopolysacchariden und Lipoteichonsäuren geeignetes chemisches modifiziertes Hohlfasermaterial aus Polyamid, Polysulfon, Polyether, Polyethylen, Polypropylen, Polyester oder Derivaten oder/und Mischungen solcher Polymere vorliegt, auf welches Tentakeln aufpolymerisiert wurden, die Anionenaustauschergruppierungen aus synthetischen oder/und halbsynthetischen oder/und natürlichen Polykationenketten in linearer oder verzweigter Form umfassen, wobei die Vorrichtung ein Totvolumen von < 150 ml und vorzugsweise ein solches von 80-130 ml aufweist.

13. Vorrichtung nach Anspruch 12, **dadurch gekennzeichnet, dass** sie als auswechselbare Filterkartusche ausgestaltet ist.

14. Vorrichtung nach Anspruch 12 oder 13, **dadurch gekennzeichnet, dass** das Hohlfasermaterial aus Nylon hergestellt ist.

15. Vorrichtung nach einem der Ansprüche 12 bis 14, **dadurch gekennzeichnet, dass** die Anionenaustauschergruppen aus Polykationenketten bestehen, welche tertiäre und/oder quartäre Amine aufweisen.

16. Vorrichtung nach einem der Ansprüche 12 bis 15, **dadurch gekennzeichnet, dass** die Anionenaustauschergruppen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- oder Di- oder Trialkylammoniumaryl-Reste, Polymere aus positiv geladenen oder tertiäre oder quartäre Aminogruppen enthaltenden Aminosäuren, wie Polylysin, Polyarginin oder Polyhistidin oder Mischpolymere oder Derivate hiervon oder Polyethylenimin sind.

17. Vorrichtung nach einem der Ansprüche 12 bis 16, **dadurch gekennzeichnet, dass** das Hohlfasermaterial ein mit Diethylaminoethylgruppen modifiziertes Polyamid ist.

18. Vorrichtung nach einem der Ansprüche 12 bis 17, **dadurch gekennzeichnet, dass** das Hohlfasermaterial eine Durchflussgeschwindigkeit von bis zu 200 ml/Minute erlaubt.

## Claims

1. Use of chemically modified hollow fiber materials for the production of an agent for the removal of bacterial lipoteichoic acids or for the collective removal of bacterial lipoteichoic acids and lipopolysaccharides from body fluids, especially blood or plasma, wherein the hollow fiber materials are selected from the group comprising polyamides, polysulfones, polyethers, polyethylene, polypropylene or polyesters and derivatives and/or mixtures of these materials, and wherein tentacles that comprise anion exchanger groups, which consist of synthetic and/or semisynthetic or/and natural polycationic chains, said chains being in linear or branched form, have been polymerized onto said hollow fiber materials.

2. Use according to claim 1, **characterized in that** hollow fibers, which were modified by graft polymerization, are used.

3. Use according to claim 1 or 2, **characterized in that** the anion exchanger groups which were polymerized onto the materials, consist of polycation chains which include tertiary and/or quarternary amines.

4. Use according to any one of claims 1 to 3, **characterized in that** the anion exchanger groups are di- or trialkylaminoalkyl, di- or trialkylaminoaryl, di- or triarylaminoalkyl, di- or triarylaminoaryl, di- or trialkylammoniumalkyl, di- or triarylammoniumalkyl, di- or triarylammoniumaryl or di- or trialkylammoniumaryl residues, polymers from amino acids that are positively charged or contain tertiary or quarternary amino groups such as polylysine, polyarginine or polyhistidine or copolymers or derivatives thereof or polyethylene-imine.

5. Use according to any one of the preceding claims, **characterized in that** the hollow fiber material is a polyamide modified with diethylaminoethyl groups.

6. Use according to any one of claims 1 to 5, **characterized in that** the agent is arranged in the form of a filter module.

7. Use according to claim 6, **characterized in that** the filter module has a dead volume of < 150 ml and preferably 80 to 130 ml.

8. Use according to claim 6 or 7, **characterized in that** the agent permits a flow rate of up to 200 ml/minute.

9. Use according to any one of claims 1 to 8, **characterized in that** the agent is designed for application in a plasma perfusion system.

10. Use according to any one of the preceding claims, **characterized in that** the agent is suitable for the treatment of a disease caused by cell wall components of gram-negative and/or gram-positive bacteria.

11. Use according to any one of the preceding claims, **characterized in that** the agent can be used within the framework of a treatment of sepsis, especially septic shock.

12. A device for removing bacterial lipoteichoic acids or bacterial lipopolysaccharides and lipoteichoic acids from blood or plasma in an extracorporeal perfusion system, **characterized in that** in a housing which is integrated into the perfusion system, there is a chemically modified hollow fiber material of polyamide, polysulfone, polyether, polyethylene, polypropylene, polyester or derivatives or/and mixtures of such polymers that is suitable for the selective removal of bacterial lipopolysaccharides and lipoteichoic acids, and onto which tentacles were polymerized comprising anion exhanger groups consisting of synthetic or/and semisynthetic or/and natural polycationic chains in linear or branched form, wherein the device has a dead volume of < 150 ml and preferably of 80-130 ml.

13. A device according to claim 12, **characterized in that** it is designed as a replaceable filter cartridge.

14. A device according to claim 12 or 13, **characterized in that** the hollow fiber material is made from nylon.

15. A device according to any one of claims 12 to 14, **characterized in that** the anion exchanger groups consist of polycation which include tertiary and/or quarternary amines.

16. A device according to any one of claims 12 to 15, **characterized in that** the anion exchanger groups are di- or trialkylaminoalkyl, di- or trialkylaminoaryl, di- or triarylaminoalkyl, di- or triarylaminoaryl, di- or trialkylammoniumalkyl, di- or triarylammoniumalkyl, di- or triarylammoniumaryl or di- or trialkylammoniumaryl residues, polymers from amino acids that are positively charged or contain tertiary or quarternary amino groups such as polylysine, polyarginine or polyhistidine or copolymers or derivatives thereof or polyethylene-imine.

17. A device according to any one of claims 12 to 16, **characterized in that** the hollow fiber material is a polyamide modified with diethylaminoethyl groups.

18. A device according to any one of claims 12 to 17, **characterized in that** the hollow fiber material permits a flow rate of up to 200 ml/minute.

## Revendications

1. Utilisation de matériaux à fibres creuses chimiquement modifiés pour la fabrication d'un moyen d'élimination des acides lipotéichoïques bactériens ou l'élimination conjointe d'acides lipotéichoïques bactériens et des lipopolysaccharides des liquides corporels, en particulier du sang ou du plasma, les matériaux à fibres creuses étant choisis dans le groupe comprenant des polyamides, des polysulfones, des polyéthers, du polyéthylène, du polypropylène ou des polyesters, ainsi que des dérivés et/ou des mélanges de ces matériaux et des tentacules ayant été greffées par polymérisation sur ces matériaux à fibres creuses, lesquelles comprennent des groupements d'échangeurs d'anions, qui sont composés de chaînes de polycations synthétiques et/ou demi-synthétiques et/ou naturels, qui se présentent sous une forme linéaire ou à ramification.

2. Utilisation selon la revendication 1, **caractérisée en ce que** l'on utilise des fibres creuses modifiées par copolymérisation greffée.

3. Utilisation selon les revendications 1 ou 2, **caractérisée en ce que** les groupements d'échangeurs d'anions greffés par polymérisation se composent de chaînes de polycations qui comportent des amines tertiaires et/ou quaternaires.

4. Utilisation selon l'une des revendications 1 à 3, **caractérisée en ce que** les groupements d'échangeurs d'anions sont des groupes di- ou trialkylaminoalkyle, di- ou trialkylaminoaryle, di- ou triarylaminoalkyle, di- ou triarylaminoaryle, di- ou triarylammoniumalkyle ou di- ou trialkylammoniumaryle, des polymères à base d'acides aminés à charge positive ou contenant des groupes amino tertiaire ou quaternaire, tels que la polylysine, la polyarginine ou la polyhistidine ou des polymères mixtes ou des dérivés de ceux-ci ou la polyéthylènimine.

5. Utilisation selon l'une des revendications précédentes, **caractérisée en ce que** le matériau à fibres creuses est un polyamide modifié par des groupes de diéthylaminoéthyle.

6. Utilisation selon l'une des revendications 1 à 5, **caractérisée en ce que** le moyen est configuré sous forme d'un module de filtration.

7. Utilisation selon la revendication 6, **caractérisée en ce que** le module de filtration présente un volume mort < 150 ml et en particulier de 80-130 ml.

8. Utilisation selon les revendications 6 ou 7, **caractérisée en ce que** le moyen permet une vitesse d'écoulement pouvant atteindre 200 ml/minute.

9. Utilisation selon l'une des revendications 1 à 8, **caractérisée en ce que** le moyen est configuré pour être appliqué dans un système de perfusion de plasma.

10. Utilisation selon l'une des revendications précédentes, **caractérisée en ce que** le moyen est approprié au traitement d'une maladie provoquée par des composants de membrane cellulaire de bactéries Gram - et/ou Gram +.

11. Utilisation selon l'une des revendications précédentes, **caractérisée en ce que** le moyen peut être utilisé dans le cadre d'un traitement de septicémie, en particulier d'un choc septique.

12. Dispositif d'élimination d'acides lipotéichoïques bactériens ou d'élimination de lipopolysaccharides et d'acides lipoteichoïques du sang ou du plasma dans un système de perfusion extra-corporel, **caractérisé en que**, dans un boîtier intégré dans le système de perfusion, un matériau à fibres creuses chimiquement modifié, qui est approprié pour l'élimination sélective de lipopolysaccharides et d'acides lipotéichoïques bactériens, est présent, le matériau étant en polyamide, polysulfone, polyéther, polyéthylène, polypropylène, polyester ou dérivés et/ou mélanges de tels polymères, des tentacules comprenant des groupements d'échangeurs d'anions en chaînes de polycations synthétiques et/ou demi-synthétique et/ou naturels, le dispositif présentant un volume mort < 150 ml, de préférence de 80-130 ml.

13. Dispositif selon la revendication 12, **caractérisé en ce qu'**il est configuré comme une cartouche de filtre échangeable.

14. Dispositif selon les revendications 12 ou 13, **caractérisé en ce que** la matière à fibres creuses est en nylon.

15. Dispositif selon l'une des revendications 12 à 14, **caractérisé en ce que** les groupements d'échangeurs d'anions se composent de chaînes de polycations qui comportent des amines tertiaires et/ou quaternaires.

16. Dispositif selon l'une des revendications 12 à 15, **caractérisé en ce que** les groupements d'échangeurs d'anions sont des groupes de di- ou trialkylaminoalkyle, di- ou trialkylaminoaryle, di- ou triarylaminoalkyle, di- ou triarylaminoaryle, di- ou trialkylammoniumalkyle, di- ou triarylammoniumalkyle, di- ou triarylammoniumaryle, di- ou trialkylammoniumaryle, des polymères à base d'acides aminés à charge positive ou contenant des groupes amino tertiaire ou quaternaire, tels que la polylysine, la polyarginine ou la polyhistidine ou des polymères mixtes ou des dérivés de ceux-ci, ou la polyéthylènimine.

17. Dispositif selon l'une des revendications 12 à 16, **caractérisé en ce que** le matériau à fibres creuses est un polyamide modifié avec des groupes de diéthylaminoéthyle.

18. Dispositif selon l'une des revendications 12 à 17, **caractérisé en ce que** le matériau à fibres creuses permet une vitesse d'écoulement pouvant atteindre 200 ml/minute.
